Europäisches Patentamt

European Patent Office

Office européen des brevets

(19)

(11) Numéro de publication: **0 146 558 B1**

(12) **FASCICULE DE BREVET EUROPEEN**

(45) Date de publication du fascicule du brevet:
**24.08.88**

(51) Int. Cl.⁴: **A 61 M 5/34,** A 61 M 5/24

(21) Numéro de dépôt: **84901733.0**

(22) Date de dépôt: **01.05.84**

(86) Numéro de dépôt international:
**PCT/CH 84/00065**

(87) Numéro de publication internationale:
**WO 84/04252 (08.11.84** Gazette **84/26)**

(54) **SERINGUE PREREMPLIE A DOSE UNITAIRE.**

(30) Priorité: **04.05.83 CH 2416/83**

(43) Date de publication de la demande:
**03.07.85 Bulletin 85/27**

(45) Mention de la délivrance du brevet:
**24.08.88 Bulletin 88/34**

(84) Etats contractants désignés:
**AT BE CH DE FR GB LI LU NL SE**

(56) Documents cités:
**EP - A - 0 111 796**
**FR - A - 714 108**
**FR - A - 2 172 101**
**US - A - 4 061 143**
**US - A - 4 365 626**

(73) Titulaire: **MEDICORP HOLDING S.A., 11 Boulevard du Prince Henri, L-2014 Luxembourg (LU)**

(72) Inventeur: **MEYER, Gabriel, 10a, chemin des Princes, CH-1222 Vésenaz (CH)**
Inventeur: **HOWALD, Ernst, 10b, chemin des Princes, CH-1222 Vésenaz (CH)**

(74) Mandataire: **Nithardt, Roland, CABINET ROLAND NITHARDT Rue Edouard Verdan 15, CH-1400 Yverdon-les-Bains (CH)**

## Description

La présente invention concerne une seringue préremplie à dose unitaire, comportant une carpule ouverte à au moins une de ses extrémités et pourvue d'une zone de section rétrécie voisine de cette extrémité, cette zone étant une partie intégrante de la carpule, un organe d'obturation engagé dans la carpule pour obturer ladite zone de section rétrécie, cet organe d'obturation étant mobile axialement entre une première position dite de stockage et une seconde position dite d'injection et comportant un conduit intérieur, une capsule distincte de l'organe d'obturation, mais liée à celui-ci et engagée sur le tronçon d'extrémité ouvert de la carpule et pourvue d'un embout équipé d'un canal axial, ce canal axial étant en communication avec le conduit intérieur de l'organe d'obturation mobile.

Ce type de seringue est décrit dans la demande de brevet européen publiée en date du 27.06.84 sous le No 0 111 796 au nom du même déposant. Ce document publié postérieurement à la date de dépôt de la présente demande constitue un art antérieur non opposable à cette demande pour l'appréciation de l'activité inventive.

Un autre type de seringue préremplie est illustré par le brevet français No 714 108 qui décrit une seringue non réutilisable comprenant une ampoule fermée à une extrémité et pourvue d'un dispositif servant à la fois d'obturateur et de vanne monté à son extrémité ouverte.

Ces seringues ne peuvent fonctionner que si le médicament est stocké dans une atmosphère en surpression et sont d'une conception compliquée et peu fiables.

Actuellement toutes les ampoules contenant des médicaments injectables sont soumises à des contrôles optiques, effectués manuellement ou automatiquement, qui sont destinés à détecter l'éventuelle présence de particules solides dans le médicament contenu dans l'ampoule. Toutefois ces contrôles s'avèrent relativement coûteux pour le fabricant et interviennent pour un pourcentage non négligeable dans le prix de revient du produit, du fait qu'ils nécessitent une station de contrôle dont les frais d'installation et de fonctionnement sont élevés.

En outre, les pertes dues au rejet des produits finis non conformes, influencent négativement le prix de revient de vente des produits conformes.

Bien que ces contrôles effectués à grands frais par le fabricant permettent théoriquement d'aboutir à un produit considéré comme acceptable, c'est-à-dire dont au moins 90 à 95% est exempt de toute particule solide supérieure à 50 µm, la réalité pratique est toute différente parce que le stockage d'une part, et les manipulations requises avant l'utilisation du produit d'autre part, peuvent en altérer considérablement la qualité. En effet, pendant le stockage certains produits peuvent former des dépôts solides qui ne sont pas entièrement dissous suite à une agitation préalable à l'injection. Dans les seringues mélangeuses, la poudre de médicament lyophilisé n'est pas toujours entièrement dissoute par le solvant de sorte que le risque d'injecter des résidus solides n'est pas inexistant. Lorsque le médicament est contenu dans une ampoule dont il convient de briser le col pour libérer le produit, des débris de verre de très faible dimension sont souvent mélangés au liquide à injecter.

Lorsque le médicament se trouve dans un récipient obturé par un bouchon en caoutchouc qu'il convient de percer avec une aiguille avant de prélever ou d'injecter le liquide médicamenteux, des particules de matière synthétique, notamment d'élastomères, peuvent se retrouver dans le liquide et risquent d'être injectées dans l'organisme du patient.

Ces problèmes évoqués ci-dessus sont bien connus par les spécialistes, mais n'ont, à ce jour, trouvé aucune solution satisfaisante globale, c'est-à-dire une solution qui permette à la fois de supprimer le contrôle coûteux effectué par le fabricant et le risque d'injecter des particules solides dans l'organisme du patient.

L'article «Glass Embolism» paru dans la revue «The Lancet» du 16 décembre 1972, page 1300, évoque le problème des particules de coton, de caoutchouc, de matériaux synthétiques, de verre ou d'autres matières solides présentes dans les doses d'injections intraveineuses, mentionne les conséquences cliniques susceptibles d'être provoquées chez le patient et propose une solution consistant à effectuer le remplissage des récipients dans une chambre sous pression.

L'article «Glass particules in intravenous injectionds» paru dans la revue «The New England journal of medicine» du 7 décembre 1972, page 1204, évoque le problème de l'introduction de particules de verre dans l'organisme humain lors d'injections intraveineuses et suggère de filtrer le médicament à l'aide d'un dispositif de filtration commercialisé par la société Millipore®. Ce dispositif comporte une pastille filtrante composée d'un boîtier contenant une membrane filtrante et pourvu d'une embouchure d'entrée et d'un embout de sortie disposés de part et d'autre de la membrane. Cette pastille est destinée à être montée entre une seringue et l'aiguille d'injection de telle manière que le liquide à injecter passe obligatoirement à travers une membrane filtrante. Son coût constitue très souvent un obstacle à son utilisation généralisée. En outre, même si son emploi est recommandée, sa mise en place peut être volontairement ou involontairement omise par le personnel soignant.

Enfin, comme cette pastille est sensée pouvoir être montée sur n'importe quelle seringue contenant n'importe quel médicament à injecter, elle doit satisfaire à des critères très larges, notamment s'adapter aux différences de volume, aux différentes viscosités et être compatible avec n'importe quel médicament.

L'article «Residues in antibiotic preparations, i: scanning electron microscopic studies of surface topography» paru dans la revue Am. J. Hosp. Pharm. 33, de May 1976 pages 433 à 443, traite du broblème des conséquences pathologiques de l'injection de particules solides présentes dans des solutions antibiotiques supposées pures selon les standards pharmaceutiques.

L'article «Particulate contamination in intravenous fluids, Nature, Origin and Hazard» paru dans la revue «Pharmaceutical Journal» du 3 mars 1973, analyse également les conséquences cliniques de l'injection de particules avec les médicaments injectés par voie intraveineuse.

L'article «Foreign bodies in contrast media for angiography» paru dans la revue Am. Hosp. Pharm. 34, de juillet 1977, pages 705–708, décrit les travaux d'analyse effectués sur des liquides destinés à des injections intra-artérielles pour des examens radiologiques, travaux qui ont révélés la présence de particules contaminantes, notamment des débris de verre, dont les dimensions vont de 0,5 µm à 450 µm. L'auteur conclut en disant que ces particules étrangères devraient être éliminées dans la mesure du possible.

L'article «Coring of rubber closures» paru dans la revue «The pharmaceutical Journal» No. 224, de février 1980, page 120, évoque le problème de la présence de particules de caoutchouc dans les solutions à injecter. L'auteur estime que ces particules ont en général leur origine dans la technique du perçage des bouchons d'obturation des ampoules contenant les produits et signale que les conséquences sur le système circulatoire du patient sont encore mal connues.

L'article «A Foreign Matter affair – The probleme of particules» paru dans la revue «The American Journal of intravenous Therapy», pages 23 à 32 résume les expériences qui ont été faites sur des animaux pour analyser les conséquences cliniques de l'injection de particules solides de coton, de verre, de caoutchouc, de matière synthétique, etc... dans le système vasculaire. L'auteur conclut son article en signalant que les causes de contamination sont multiples et que, malgré les efforts déjà entrepris, un progrès technique s'impose pour tenter d'amener la pureté effetive des médicaments injectés, le plus près possible de 100%.

Tous les articles de la littérature médicale démontrent clairement que la présence de particules solides de toute nature dans les injectables, constitue un danger non négligeable pour le malade, et qu'actuellement aucun moyen économiquement valable n'a permis de supprimer ce risque sans accroître exagérément le coût des soins dispensés.

La présente invention se propose de pallier tous les inconvénients susmentionnés en proposant un système permettant de supprimer le contrôle coûteux traditionnellement effectué chez le fabricant, évitant toute contamination du médicament à injecter par des particules solides de verre ou de caoutchouc habituellement introduites lors des manipulations précédant l'injection et garantissant l'absence, dans le liquide effectivement injecté de toute bulle d'air et de toute particule solide, quelque soit son origine, dont les dimensions sont supérieures à une dimension déterminée.

Dans ce but, la seringue selon l'invention telle que définie en préambule comprend également au moins un micro-filtre à membrane disposé soit entre le conduit intérieur de l'organe d'obturation mobile et le canal axial de l'embout, soit dans ledit canal axial, soit dans ledit canal axial et dans ledit conduit intérieur, au moins deux micro-filtres étant prévus dans ce dernier cas.

On pourrait objecter qu'il existe déjà des seringues contenant un filtre, telles que par exemple celles décrites par le brevet américain No. 4 365 626. Ces seringues se composent en fait d'une ampoule dont les extrémités sont obturées par deux bouchons en élastomère, et d'un embout porte-aiguille équipé d'une double aiguille dont l'une est destinée à percer l'un des bouchons d'obturation de l'ampoule, et d'un filtre dont le but est d'arrêter les particules de caoutchouc arrachées lors du percement du bouchon. Toutefois, on notera que le filtre est solidaire de l'embout porte-aiguille monté sur l'ampoule juste avant l'utilisation de la seringue, qu'il constitue de ce fait un élément accessoire pouvant éventuellement être remplacé par une autre aiguille non munie de filtre, et qu'il ne remplit pas son rôle sur le plan de la garantie totale de l'absence de particules solides à l'injection, évoqué ci-dessus. En outre, le filtre décrit, logé dans une structure de support complexe portant également l'aiguille de percement du bouchon d'obturation, a essentiellement comme but d'éliminer la contamination engendrée lors de ce percement. Enfin, vu le nombre de pièces composant le système, la seringue décrite est d'un coût de revient tellement élevé qu'il ne saurait être question de l'imposer comme un système universel pour les pays industrialisés et certainement pas adaptable aux conditions médicales rencontrées dans certains pays économiquement peu favorisés.

On pourrait aussi objecter qu'il existe des seringues équipées d'un filtre dit «en profondeur», telles que celles décrites par le brevet français publié sous le No. 2 361 121 comprenant un corps de seringue pourvu d'un piston et obturé à une extrémité par une membrane d'obturation. Le capuchon porte-aiguille est équipé d'un filtre de profondeur destiné, comme précédemment, à arrêter les particules éventuellement arrachées à la membrane en élastomère au cours de son percement.

Les filtres en profondeur sont habituellement constitués par une sorte de tampon réalisé au moyen de divers matériaux tels que par exemple du métal fritté ou tout autre matière appropriée, qui définit un ensemble de micro-canaux dont la section est sensée être suffisamment faible pour arrêter les particules solides indésirables. Néanmoins, les filtres en profondeur ne permettent pas de garantir une limite inférieure de filtration à 100%. En effet, si des essais statistiques permettent d'affirmer que tel type de filtre en profondeur filtre par exemple à 80% toutes les particules dont la section est égale ou supérieure à 50 µm, ceci n'exclut pas la possibilité que quelques particules de section bien supérieure, par exemple égales à 150 µm puissent trouver un passage et se retrouver en fin de compte dans le liquide filtré.

Les micro-filtres à membrane, par contre, permettent de garantir d'une manière absolue la filtration à 100% de toute particule solide dont le diamètre est supérieur à la dimension nominale des pores du filtre. La section des pores est définie par le fabricant et peut être contrôlée par la technique connue dite «du point de bulle». Le micro-filtre à membrane est équivalent à un tamis dont les ouvertures sont toutes identiques et définies par construction.

Selon un mode de réalisation préféré, le micro-filtre à membrane de la seringue préremplie selon l'invention présente des pores dont la plus grande dimension est comprise entre 0,2 et 5 µm.

Les matériaux utilisés pour la réalisation de ce micro-filtre sont de préférence choisis parmi les substances suivantes: les polyamides, les acryliques copolymères enrobés de polyamide non tissé, les esters de cellulose, le polytétrafluoréthylène, les polypropylènes.

La présente invention sera mieux comprise en référence à la description de diverses variantes et aux dessins annexés dans lesquels:

La figure 1 représente une vue en coupe d'une seringue à piston selon l'invention,

la figure 2 représente une vue en coupe partielle d'une première variante d'une seringue à piston telle que représentée par la fig. 1,

la figure 3 représente une vue partielle d'une seringue selon l'invention illustrant essentiellement sa capsule munie d'un filtre et d'un organe de verrouillage,

la figure 4 représente une variante de la seringue représentée par la fig. 3,

la figure 5 représente une autre forme de réalisation dans laquelle le médicament à injecter se trouve dans une carpule sous pression,

la figure 6 représente une autre forme de réalisation dans laquelle le médicament à injecter se trouve dans une carpule fermée à une extrémité, l'organe d'obturation jouant le rôle de piston,

la figure 7 illustre une variante du système de la fig. 6,

la figure 8 illustre un autre mode de fixation de l'embout de raidissement du système de la fig. 7,

la figure 9 représente une variante de la réalisation selon la fig. 8, et

les figures 10 et 11 illustrent différentes possibilités de montage du filtre par un procédé de surinjection.

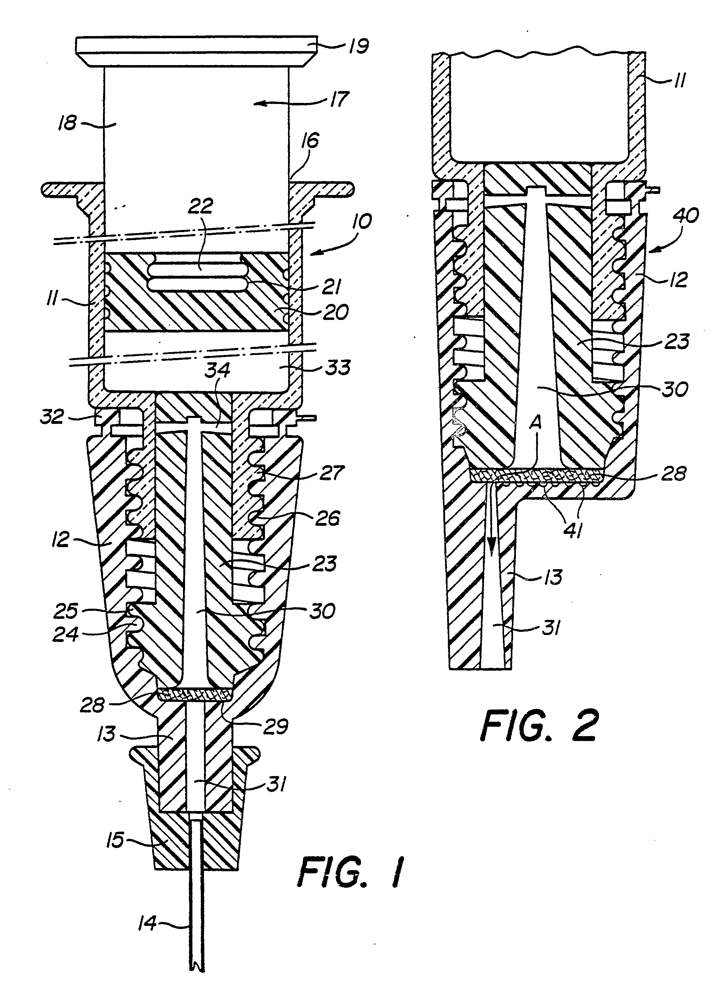

En référence à la fig. 1, la seringue 10 représentée, comporte une carpule 11, une capsule 12 adaptée à l'extrémité inférieure de la carpule 11 et pourvue d'un embout 13 de fixation d'une aiguille d'injection 14 (illustrée partiellement) portée par un porte-aiguille 15 engagé sur l'embout 13. La carpule 11 de la seringue présente, dans ce cas, une ouverture supérieure 16 traversée par un piston 17 constitué par une tige de piston 18, un poussoir 19 et le piston proprement dit 20. Ce piston 20 présente au centre de sa face supérieure, un évidement 21 qui coopère avec un embout cannelé 22 ménagé à l'extrémité inférieure de la tige de piston 18, pour assurer la liaison entre la tige de piston 18 et le piston 20. Il est bien entendu que la carpule 11 ouverte à ses deux extrémités, pourrait être remplacée par une carpule obturée à son extrémité supérieure dans laquelle la dose unitaire de médicament se trouve contenue sous pression.

La zone de section rétrécie ou col 27 de la carpule est obturée par un organe d'obturation mobile 23 rendu solidaire de la capsule 12 par vissage ou tout autre moyen adéquat. A cet effet, la capsule comporte un filetage 24 conçu pour s'ajuster à un filetage 25 complémentaire de la base de l'organe d'obturation 23.

La capsule 12 a la forme générale d'un capuchon dont la paroi intérieure présente un filetage 26 qui permet son vissage sur le col fileté 27 de la carpule 11 de la seringue. Entre l'extrémité inférieure de l'organe d'obturation mobile 23 et le fond de la capsule 12, est ménagé un micro-filtre à membrane 28 retenu par un épaulement 29 et interposé entre le conduit intérieur 30 de l'organe d'obturation 23 et le canal axial 31 traversant l'embout 13 et communiquant avec l'aiguille d'injection 14. Le micro-filtre à membrane est avantageusement conçu pour arrêter toute particule dont une dimension est supérieure à 2 µm. Un organe de verrouillage 32 constitué, dans ce cas, par une languette solidaire du rebord supérieur de la capsule 12, bloque cette capsule par rapport à la carpule 11, dans sa position d'obturation représentée par la figure. Dans cette position, le médicament est contenu dans la chambre 33 de la carpule 11. Cette chambre est fermée à sa base par l'organe d'obturation 23.

On notera que l'organe d'obturation 23 est lié à la capsule 12 et que le micro-filtre à membrane 28 est monté à demeure entre la base de cet organe d'obturation et le fond de cette capsule. Le micro-filtre à membrane 28 ne se trouve pas en contact avec le médicament pendant le stockage de la seringue.

Lorsque le soignant veut injecter le médicament à son patient, il arrache la languette de verrouillage 32, visse la capsule 12 à fond sur le col 27 de la carpule 11 de la seringue, ce qui a pour effet de faire pénétrer l'organe d'obturation 23 dans la chambre 33, d'une manière suffisante pour que les embouchures de la branche transversale 34 du conduit intérieur 30 en forme de T, soient localisées à l'intérieur de cette chambre. Le médicament peut s'écrouler à travers ce conduit en traversant obligatoirement le micro-filtre à membrane 28 et pénètre par l'intermédiaire du canal axial 31 dans l'aiguille d'injection 14, sous l'effet de la pression exercée par le piston 20.

La fig. 2 représente partiellement une seringue 40 dont la configuration est sensiblement identique à celle de la fig. 1. Elle comporte comme la précédente une carpule 11 ainsi qu'une capsule 12 contenant un organe d'obturation 23.

Dans cet exemple, l'embout 13 est décentré de sorte que le canal axial 31 n'est plus comme précédemment disposé dans le prolongement du conduit intérieur 30 ménagé à travers l'organe d'obturation 23. Le micro-filtre à membrane 28 a, dans ce cas, en plus de son rôle habituel de filtre, la fonction de raccorder les deux conduits 30 et 31 pour permettre l'écoulement du médicament contenu initialement dans la carpule 11 de la seringue 40, dans le sens de la flèche A. Pour permettre l'écoulement du liquide filtré en direction du canal axial 31, le fond de la capsule 12 est équipé d'une série de canaux de drainage 41 qui collectent le liquide vers le canal 31.

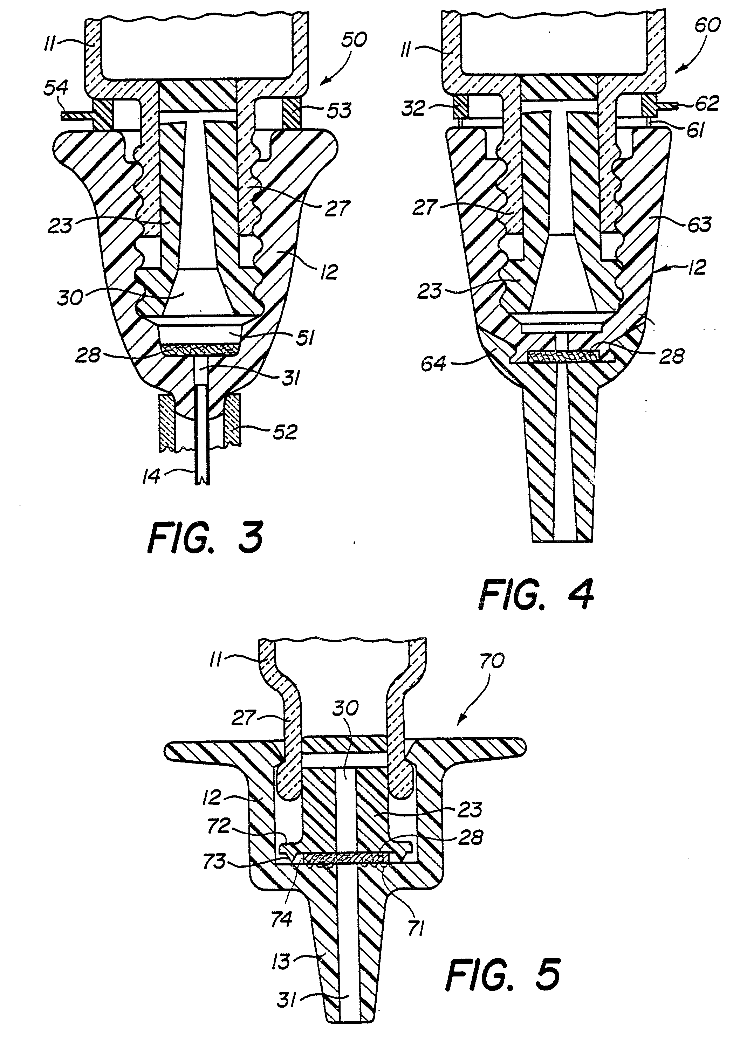

La fig. 3 représente une vue partielle d'une seringue préremplie à dose unitaire 50 comportant comme précédemment une carpule 11, une capsule 12 vissée sur le col 27 de la carpule 11 et équipée d'un organe d'obturation 23 pourvu d'un conduit intérieur 30 en forme de T.

Dans cet exemple, le micro-filtre à membrane 28 est logé librement dans une cavité 51 ménagée à la base de la capsule 12. Sous le micro-filtre à membrane 28, prend naissance le canal axial 31 qui est, dans ce cas, conçu pour recevoir directement l'extrémité supérieure d'une aiguille d'injection 14, collée ou fixée par tout autre moyen approprié, et protégée par un capuchon tubulaire 52 adapté sur un embout de la capsule 12.

Le micro-filtre à membrane 28 est avantageusement réalisée en un matériau ayant approximativement le même point de fusion que la matière de la capsule 12, ce qui permet de réaliser une soudure périphérique dudit filtre contre le fond de la capsule.

L'organe de verrouillage, destiné à bloquer initialement la capsule dans sa position d'obturation est, dans ce cas, constitué par une bague 53 rapportée, c'est-à-dire qui n'est pas liée directement à la capsule. Cette bague 53 peut être constituée par une bande repliée en anneau et dont les extrémités sont fixées l'une contre l'autre par soudure, enclipsage, collage, etc. . . Cette bague rapportée 53 pourrait également être remplacée par une languette liée en continu ou par des points d'ancrage à la capsule 12. Elle pourrait également être équipée d'une patte d'arrachage 54.

Comme précédemment, le micro-filtre à membrane 28 est lié de façon définitive à l'ensemble organe d'obturation mobile 23 — capsule 12 et interposé entre le conduit intérieur 30 et le canal axial 31.

La fig. 4 illustre une seringue 60 pourvue d'une carpule 11 et d'une capsule 12 vissée sur le col 27 de la carpule. Un organe d'obturation 23 identique à celui de la seringue 50 représentée par la fig. 3, équipe la capsule 12. Un languette 32 reliée au bord supérieur de la capsule 12 par des points d'ancrage 61 et pourvue d'une patte d'arrachage 62, sert d'organe de verrouillage et bloque la capsule 12 dans sa position d'obturation.

Dans cet exemple, la capsule 12 est en réalité composée de deux éléments 63 et 64 emboîtables assemblés au moment du montage. Un micro-filtre à membrane 28, logé dans un évidement spécialement ménagé à cet effet, est pincé entre les deux éléments 63 et 64. Ce micro-filtre 28 est obligatoirement traversé par le médicament au moment de l'injection.

La fig. 5 illustre une autre forme de réalisation d'une seringue préremplie à dose unitaire 70 comportant une carpule 11 constituée par une ampoule en verre ou en matière synthétique fermée à son extrémité supérieure, une capsule 12 et un organe d'obturation 23 engagé dans le col 27 de la carpule 11. La capsule 12 est équipée d'un embout porte-aiguille 13 traversé par un canal axial 31 disposé sensiblement dans le prolongement du conduit intérieur 30 de l'organe d'obturation. Un micro-filtre à membrane 28 est interposé entre le conduit intérieur 30 et le canal axial 31. Une grille de drainage 71 équipe le fond de la capsule pour faciliter l'écoulement du liquide médicamenteux filtré.

Dans cet exemple, l'organe d'obturation 23 comporte un rebord annulaire 72 pourvu d'une protubérance 73 de section sensiblement triangulaire destinée à se loger dans un évidement annulaire 74 ménagé dans la base de la capsule 12. Cet agencement permet de souder l'organe d'obturation 23 à la capsule 12 au moyen d'une sonotrode, de maintenir le filtre en position et d'assurer l'étanchéité autour de ce filtre de telle manière que le liquide initialement contenu dans la carpule passe obligatoirement à travers le micro-filtre à membrane 28.

Par contre, étant donné que le micro-filtre à membrane est imperméable au gaz lorsqu'il est mouillé et lorsque ce gaz se trouve à une pression inférieure au point de bulle caractérisant ce filtre, le médicament à injecter peut être stocké à l'intérieur de la carpule sous une pression telle qu'en fin d'injection, le gaz reste à une pression inférieure à ce point de bulle de sorte qu'il ne risque pas d'être injecté dans le tissu du patient.

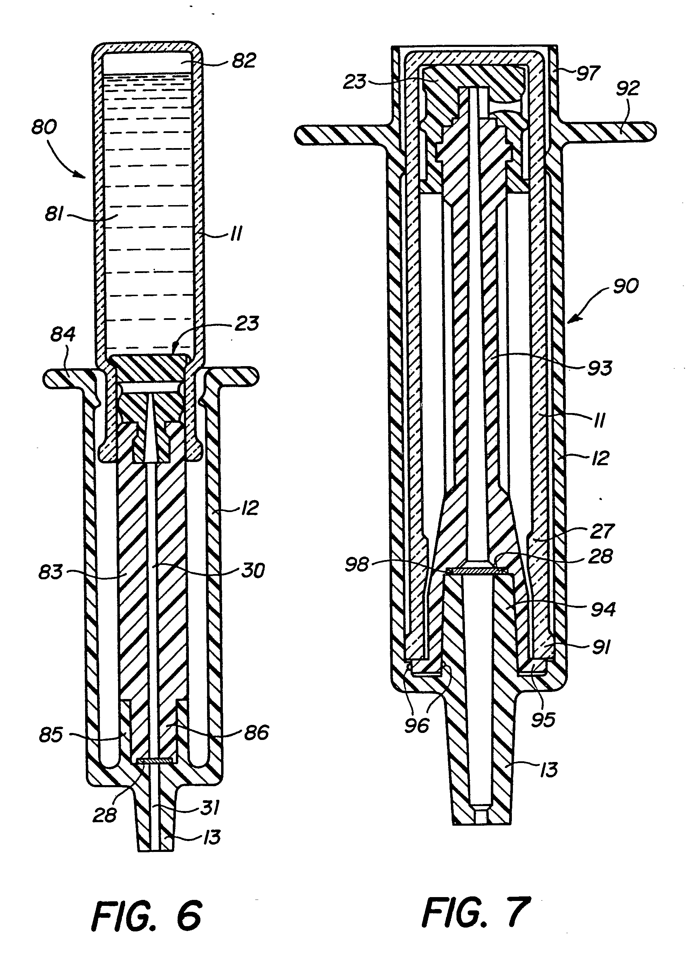

La fig. 6 représente une autre forme de réalisation d'une seringue préremplie 80 à dose unitaire 81 contenue dans une carpule 11 sous la très légère surpression d'un gaz 82. La carpule 11 est obturée par un organe d'obturation 23 fixé à l'extrémité libre d'une tige 83 solidaire du fond de la capsule 12 munie à son extrémité supérieure de deux ailettes 84. La fixation de l'organe d'obturation 23 à l'extrémité de la tige 83 s'effectue d'une manière quelconque, pourvue que cette liaison soit rigide pendant le fonctionnement de la seringue. Pour permettre la mise en place du micro-filtre à membrane 28, la base de la capsule 12 est pourvue d'une embase annulaire 85 dans laquelle s'emboîte, au moment du montage, un téton 86 légèrement conique formant l'extrémité inférieure de la tige 83. Comme dans les exemples précédents, le micro-filtre à membrane 28 est interposé entre le conduit intérieur 30 de l'organe d'obturation 23 et le canal axial 31 de l'embout porte-aiguille 13.

Dans cette seringue, l'organe d'obturation joue le rôle d'un piston lorsque l'opérateur appuie sur le fond de la carpule pour repousser cette dernière en direction de la capsule.

Une variante de ce dispositif est illustrée par la fig. 7 représentant la seringue 90 dans sa position de fin d'injection. Elle comporte une carpule 11 pourvue d'une zone de section rétrécie ou col 27 et d'un rebord 91 entourant son ouverture, une capsule 12 munie d'ailettes de maintien 92 et d'un embout porte-aiguille 13 traversé par un canal axial 31, et un organe d'obturation 23 comportant en fait un bouchon d'obturation en élastomère porté à l'extrémité d'un embout de raidissement 93 rendu solidaire de la base de la capsule 12 par tout autre moyen approprié. Dans l'exemple représenté, l'extrémité inférieure de l'embout de raidissement 93 s'emboîte sur une embase tronconique 94 solidaire du fond de la capsule 12. Cette embase sert de support au micro-filtre à membrane 28 qui est pincé entre les surfaces en regard de l'embout de raidissement 93 et de l'embase porte-filtre 94. Le rebord annulaire 95 de l'embout de raidissement 93 peut être fixé au fond de la capsule 12 par simple serrage, mais peut également comporter un bourrelet d'encliquetage 96 qui coopère avec un évidement annulaire complémentaire ménagé autour de l'embase 94.

Comme le montre clairement cette figure, lorsque la carpule 11 est complètement enfoncée dans la capsule 12, son fond affleure le rebord annulaire 87 de l'extrémité supérieure de la capsule 12, ce qui garantit sa non-réutilisation du fait qu'il est impossible d'extraire la carpule pour la ramener dans sa position initiale sans endommager le système.

Cette conception constitue un complément à l'effet de neutralisation en fin d'injection assuré par le micro-filtre à membrane qui, à l'état mouillé, interdit toute entrée d'air et, par conséquent, tout retour de la carpule dans sa position initiale.

Pour assurer une meilleure retenue du micro-filtre à membrane sur l'embase porte-filtre 94, celle-ci peut être équipée de pointes 98 destinées à pincer le filtre et empêcher toute fuite par capillarité du liquide médicamenteux.

Le rebord 95 pourrait également présenter un autre profil adapté à un profil complémentaire de la paroi intérieure de la capsule, en particulier tout profil se prêtant à une soudure par sonotrode.

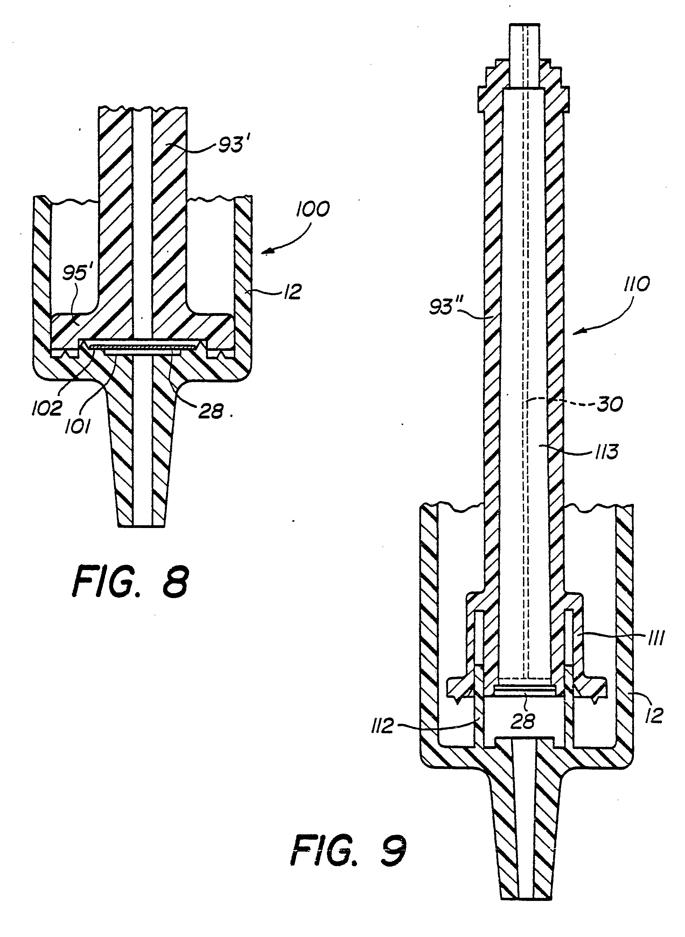

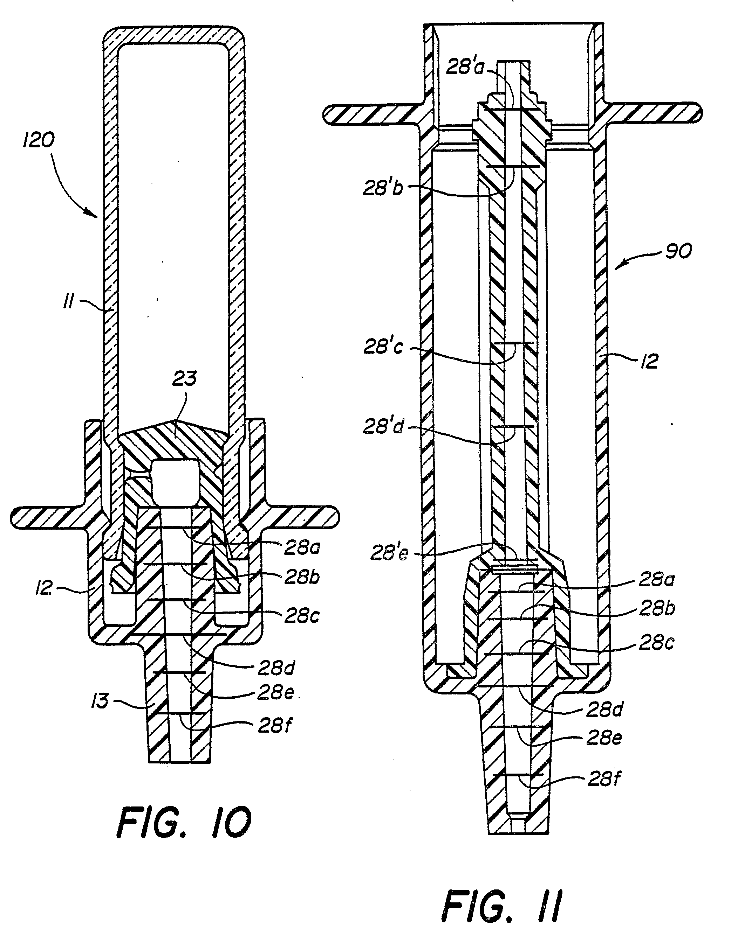

Toutes sorte de variantes peuvent être imaginées, notamment celles illustrées par les figures 9 et 10. Dans l'exemple de seringue 100 illustrée partiellement par la fig. 8, l'embout de raidissement 93' comporte un rebord annulaire 95' destiné à être lié par une soudure aux ultra-sons contre le fond de la capsule 12. Le micro-filtre à membrane 28 est contenu dans une cavité 101 ménagée entre la base de l'embout de raidissement 93' et le fond de la capsule 12, et est pincé sur son pourtour entre le rebord annulaire 95' et un bourrelet 102 ménagé au fond de la capsule. Cette technique permet d'assurer une étanchéité parfaite et de retenir les éventuelles fibres libres du filtre.

Dans l'exemple de seringue 110 illustrée partiellement par la fig. 9, l'embout de raidissement 93'' comporte une base en forme de cloche 111 qui s'ajuste sur une embase annulaire 112 solidaire du fond de la capsule 12. L'organe de raidissement contient un noyau 113 traversé par le conduit intérieur 30 et dont l'extrémité inférieure porte le micro-filtre à membrane 28. Le filtre est serré entre deux surfaces planes en regard lorsque la cloche 111 est ajustée sur l'embase 112. La cloche 111 peut comporter un rebord annulaire à être soudé contre le fond de la capsule 12.

Dans les exemples précédents, le micro-filtre à membrane 28 a toujours été positionné entre deux pièces fabriquées séparément puis assemblées par diverses techniques. La technique de la surinjection permet de mettre en place ce filtre à n'importe quel endroit transversalement par rapport au conduit intérieur 30 ou au canal axial 31.

La fig. 10 illustre partiellement une seringue préremplie à dose unitaire 120, dans laquelle le liquide à injecter est contenu dans une carpule à gaz sous pression (non représentée). Les positions possibles du micro-filtre à membrane sont indiquées par les références 28a, 28b, 28c, 28d, 28e, 28f. Le filtre découpé dans la machine à injecter est tenu par deux tiges. Par surinjection il est intégré à l'embout de raidissement de l'organe d'obturation 23 solidaire de la capsule 12 ou de l'embout porte-aiguille 13 également solidaire de la capsule 12.

La fig. 11 illustre partiellement la seringue préremplie à dose unitaire 90 identique à celle décrite en référence à la fig. 8. Les références 28a, 28b, 28c, 28d, 28 e et 28f représentent comme précédemment les positions possibles du micro-filtre à membrane 28 intégré à l'embase 94 par un procédé de surmoulage. Les références 28'a, 28'b, 28'c, 28'd, 28'e, 28'f illustrent les positions possibles du micro-filtre à membrane 28 intégré à l'embout de raidissement par surmoulage.

On constate que dans toutes les variantes décrites, le micro-filtre à membrane a une surface utile inférieure à la plus petite section droite de la carpule. Dans la pratique, le matériau filtrant utilisé pour la confection du micro-filtre à membrane et le lchoix du diamètre de ses pores est déterminé par le type, la viscosité et le volume du médicament contenu dans la carpule, ce qui permet d'adapter de manière absolument spécifique, donc économique et rentable, le filtre au médicament.

D'autres variantes seraient imaginables. Il est cependant essentiel que la construction de ces seringues se prête à une fabrication aisée avec mise en place automatique de tous les composants.

**Revendications**

1. Seringue préremplie à dose unitaire, comportant une carpule (11) ouverte à au moins une de ses extrémités et pourvue d'une zone de section rétrécie (27) voisine de cette extrémité, cette zone étant une partie intégrante de la carpule (11), un organe d'obturation (23) engagé dans la carpule pour obturer ladite zone de section rétrécie (27), cet organe d'obturation étant mobile axialement entre une première position dite de stockage et une seconde position dite d'injection et comportant un conduit intérieur (30), une capsule (12) distincte de l'organe d'obturation (23), mais liée à celui-ci et engagée sur le tronçon d'extrémité ouvert de la carpule (11), et pourvue d'un embout (13) équipé d'un canal axial (31), ce canal axial étant en communication avec le conduit intérieur (30) de l'organe d'obturation mobile (23), ainsi qu'au moins un micro-filtre à membrane (28, 28a, b, c, d, e, f, 28′a, b, c, d, e,) disposé soit entre le conduit intérieur (30) de l'organe d'obturation mobile (23) et le canal axial (31) de l'embout (13), soit dans ledit canal axial (31), soit dans ledit canal axial (31) et dans ledit conduit intérieur (30), au moins deux micro-filtres étant prévus dans ce dernier cas.

2. Seringue selon la revendication 1, caractérisée en ce que le micro-filtre à membrane (28) comporte des pores dont la dimension maximale est comprise entre 0,2 et 5 µm.

3. Seringue selon la revendication 1, caractérisé en ce que le micro-filtre à membrane (28) est constitué par une substance choisie parmi le groupe suivant: les polyamides, les acryliques copolymères enrobés de polyamide non tissé, les esters de cellulose, le polytétrafluoréthylène, les polypropylènes.

4. Seringue selon la revendication 1, caractérisée en ce que le micro-filtre à membrane (28) est pincé entre l'extrémité inférieure de l'organe d'obturation (23) et le fond de la capsule (12).

5. Seringue selon la revendication 1, caractérisée en ce que le micro-filtre à membrane (28) est logé dans une cavité (101) ménagée dans le fond de la capsule (12) et en ce qu'il est rendu solidaire de ce fond par collage ou soudure.

6. Seringue selon les revendications 1 ou 5, caractérisée en ce que le micro-filtre à membrane (28) et la capsule (12) sont réalisés en des matériaux ayant approximativement le même point de fusion et en ce que le filtre est fixé au fond de la capsule par une soudure périphérique.

7. Seringue selon la revendication 1, caractérisée en ce que l'organe d'obturation (23) se compose d'un bouchon d'obturation (23) et d'un embout de raidissement (83, 93), et en ce que le micro-filtre à membrane (28) est disposé entre l'extrémité de l'embout de raidissement (83, 93) et le fond de la capsule.

8. Seringue selon la revendication 7, caractérisée en ce que le micro-filtre à membrane (28) est pincé entre l'embout de raidissement (93) et le fond de la capsule (12).

9. Seringue selon la revendication 7, caractérisée en ce que l'embout de raidissement (93) est soudé au fond de la capsule, en ce que le micro-filtre à membrane (28) est logé dans une cavité (101) ménagée entre l'embout de raidissement et le fond de la capsule (12) et en ce que ce micro-filtre à membrane (28) est pincé entre un bord plan annulaire (95′) de l'embout de raidissement (93) entourant ladite cavité (101) et une protubérance annulaire (102) du fond de la capsule (12).

10. Seringue selon la revendication 7, caractérisée en ce que le fond de la capsule (12) comporte une embase annulaire (85) au fond de laquelle est disposé le micro-filtre à membrane (28) et en ce que l'embout de raidissement (83) comporte un téton (86) légèrement conique qui s'emboîte dans ladite embase.

11. Seringue selon la revendication 7, caractérisée en ce que le fond de la capsule comporte une embase annulaire (112) disposée dans le prolongement de l'embout porte-aiguille à l'intérieur de la capsule, en ce que l'embout de raidissement comporte une base en forme de cloche (111) qui s'emboîte sur ladite embase annulaire (112) et ce que le micro-filtre à membrane (28) est disposé entre ladite embase et ladite base en forme de cloche.

12. Seringue selon l'une quelconque des revendications 1, 7 ou 11, caractérisée en ce que le micro-filtre à membrane (28) est intégré par surmoulage au fond de la capsule, à l'embout porte-aiguille, à l'embout de raidissement ou l'embase annulaire solidaire du fond de la capsule.

13. Seringue selon la revendication 1, caractérisée en ce que le médicament à injecter est soumis à la pression d'un gaz contenu dans la carpule fermée à une de ses extrémités, et en ce que en fin d'injection, le gaz au moins partiellement détendu occupe tout le volume compris entre les parois intérieurs de la carpule (11) et le micro-filtre à membrane (28).

14. Seringue selon l'une quelconque des revendications précédentes, caractérisée en ce que la hauteur intérieure de la capsule (12) est au moins égale à la hauteur extérieure de la carpule (11).

15. Seringue selon l'une quelconque des revendications précédentes, caractérisée en ce que la surface utile du filtre est inférieure à la plus petite section droite de la carpule (11).

**Claims**

1. Prefilled single dose syringe comprising an ampoule (11) open at at least one of its ends and provided with a narrowed-diameter portion (27) neighboring this end, this portion being integral with the ampoule (11), a stopper means (23) fitting into the ampoule to stopper the said narrowed-diameter portion (27), this stopper means being movable axially between a first position called the storage position and a second position called the injection position and comprising an internal conduit (30), a capsule (12) distinct from the stopper means (23) but attached to this stopper means and fitted on the part of open end of the ampoule (11) and provided with a tip (13) provided with an axial channel (31), this axial channel being intented to communicate with the internal conduit (30) of the movable stopper means (23), and at least one membrane microfilter (28; 28a, b, d, d, e, f; 28′a, b, c, d, e) located either between the internal conduit (30) of the movable stopper means (23) and the axial channel (31) of the tip (13), or inside said axial channel (31), or inside said axial channel (31) and said internal conduit (30), at least two microfilters being provided in this last case.

2. Syringe according to claim 1, characterized in that the membrane microfilter (28) comprises pores with a maximum diameter of between 0.2 and 5 µm.

3. Syringe according to claim 1, characterized in that the membrane microfilter (28) is made of a substance selected from the following groups: polyamides, acrylic copolymers coated with nonwoven polyamide, cellulose esters, polytetra-fluorethylene, polypropylenes.

4. Syringe according to claim 1, characterized in that the membrane microfilter (28) is pressed between the lower end of the stopper means (23) and the bottom of the capsule (12).

5. Syringe according to claim 1, characterized in that the membrane (28) is located in a hollow (101) cut into the bottom of the capsule (12) and in that it is attached to this bottom by gluing or welding.

6. Syringe according to claims 1 or 5, characterized in that the membrane microfilter (28) and the capsule (12) are made of materials having approximately the same melting point ant in that the filter is attached to the bottom of the capsule by peripheral welding.

7. Syringe according to claim 1, characterized in that the stopper means (23) comprises a stopper plug (23) and a tightening tip (83, 93) and in that the membrane microfilter (28) is located between the end of the tightening tip (83, 93) and the bottom of the capsule (12).

8. Syringe according to claim 7, characterized in that the membrane microfilter (28) is pressed between the tightening tip (93) and the bottom of the capsule (12).

9. Syringe according to claim 7, characterized in that the tightening tip (93) is welded to the bottom of the capsule, in that the membrane microfilter (28) is located in a hollow (101) cut between the tightening tip and the bottom of the capsule (12) and in that this membrane microfilter (28) is pinched between a planar, ring-shaped rim (95') of the thightening tip (93') surrounding said hollow (101) and a ring-shaped protuberance (102) of the bottom of the capsule (12).

10. Syringe according to claim 7, characterized in that the bottom of the capsule (12) comprises a ring-shaped base (85), at the bottom of which the membrane microfilter (28) is placed, and in that the tightening tip (83) comprises a slightly conical stud that fits into said base.

11. Syringe according to claim 7, characterized in that the bottom of the capsule comprises a ring-shaped base (112) located in the extension of the needle-carrying tip inside the capsule, in that the tightening tip comprises a base shaped like a bell (111) that fits over said ring-shaped base (112), and in that the membrane microfilter (28) is located between said base and said base shaped like a bell.

12. Syringe according to any one of claims 1, 7 or 11, characterized in that the membrane microfilter (28) is integrated through moulding with the bottom of the capsule, the needle-carrying tip, the tightening tip, or the ring-shaped base that is attached to the bottom of the capsule.

13. Syringe according to claim 1, characterized in that the medicine be injected is put under the pressure of a gas contained in the ampoule closed at one of its ends, and in that at the end of the injection, the gas, at least partially expanded, fills the entire volume between the inner walls of the ampoule (11) and the membrane microfilter (28).

14. Syringe according to any one of the preceding claims, characterized in that the inner height of the capsule (12) is at least equal to the outer height of the ampoule (11).

15. Syringe according to any one of the preceding claims, characterized in that the useful surface of the filter is less than the smallest section diameter of the ampoule (11).

## Patentansprüche

1. Mit Einheitsdosis vorgefüllte Spritze, die aus folgenden Elementen besteht: einer mindestens an einem ihrer Enden offenen und in der Nähe dieses Endes mit einer Zone von eingeschnürtem Querschnitt (27) versehenen Ampulle (11), wobei diese Zone ein wesentlicher Bestandteil der Ampulle (11) ist, einem in die Ampulle eingesetzten Verschlussorgan (23), um die Zone mit eingeschnürtem Querschnitt (27) zu verschliessen, dabei ist dieses einen inneren Kanal (30) aufweisende Verschlussorgan axial zwischen einer ersten sogenannten Einlagerungsstellung und einer zweiten sogenannten Einspritzstellung beweglich, einer vom Verschlussorgan (23) getrennten aber mit ihm verbundenen auf das offene Endstück der Ampulle (11) gesetzten und mit einem Ansatz (13), der eine axiale Bohrung (31) hat, versehenen Kapsel (12), wobei diese axiale Bohrung mit dem inneren Kanal (30) des beweglichen Verschlussorganes (23) in Verbindung steht, sowie mindestens einem membranartigen Mikrofilter (28, 28a, b, c, d, e, f, 28'a, b, c, d, e) das entweder zwischen dem inneren Kanal (30) des beweglichen Verschlussorgans (23) und der Axialbohrung (31) des Ansatzes (13), oder in der Axialbohrung (31), oder in der Axialbohrung (31) und in dem inneren Kanal (30) angeordnet ist, wobei in letzterem Fall mindestens zwei Mikrofilter vorgesehen sind.

2. Spritze nach Anspruch 1, dadurch gekennzeichnet, dass das membranartige Mikrofilter (28) Poren aufweist, deren maximale Abmessung zwischen 0,2 und 5 μm liegt.

3. Spritze nach Anspruch 1, dadurch gekennzeichnet, dass das membranartige Mikrofilter (28) aus einer Substanz besteht, die aus nachstehender Gruppe ausgewählt ist: Polyamide, nicht gewebte, mit Polyamid beschichtete Acrylcopolymere, Celluloseester, Polytetrafluoräthylen, Polypropylene.

4. Spritze nach Anspruch 1, dadurch gekennzeichnet, dass das membranartige Mikrofilter (28) zwischen das untere Ende des Verschlussorgans (23) und den Boden der Kapsel (12) geklemmt ist.

5. Spritze nach Anspruch 1, dadurch gekennzeichnet, dass das membranartige Mikrofilter (28) in einer Vertiefung (101) liegt, die sich am Boden der Kapsel (12) befindet und dass es mit diesem Boden durch Kleben oder Schweissen fest verbunden ist.

6. Spritze nach Anspruch 1 oder 5, dadurch gekennzeichnet, dass das membranartige Mikrofilter (28) und die Kapsel (12) aus Werkstoffen hergestellt sind, die ungefähr denselben Schmelzpunkt haben und dass das Filter am Boden der Kapsel durch eine Umfangsschweissung befestigt ist.

7. Spritze nach Anspruch 1, dadurch gekennzeichnet, dass das Verschlussorgan (23) aus einem Verschlussstopfen (23) und einem Versteifungsansatz (83, 93) besteht, und dass das membranartige Mikrofilter (28) zwischen dem Ende des Versteifungsansatzes (83, 93) und dem Boden der Kapsel (12) angeordnet ist.

8. Spritze nach Anspruch 7, dadurch gekennzeichnet, dass das membranartige Mikrofilter (28) zwischen dem Versteifungsansatz (93) und dem Boden der Kapsel (12) eingeklemmt ist.

9. Spritze nach Anspruch 7, dadurch gekennzeichnet, dass der Versteifungsansatz (93') an den Boden der Kapsel geschweisst ist, dass das membranartige Mikrofilter (28) in einer zwischen dem Versteifungsansatz und dem Boden der Kapsel (12) vorgesehenen Vertiefung (101) untergebracht ist und dass dieses membranartige Mikrofilter (28) zwischen einem die Vertiefung (101) umgebenden ringförmigen Rand (95') des Versteifungsansatzes (93') und einem ringförmigen Vorsprung (102) am Boden der Kapsel (12) festgeklemmt ist.

10. Spritze nach Anspruch 7, dadurch gekennzeichnet, dass der Boden der Kapsel (12) einen ringförmigen Sitz (85) aufweist, auf dessen Boden das membranartige Mikrofilter liegt und dass der Versteifungsansatz (83) einen leicht konischen Zapfen (86) aufweist, der in den ringförmigen Sitz eingeschoben ist.

11. Spritze nach Anspruch 7, dadurch gekennzeichnet, dass der Boden der Kapsel einen ringförmigen Sitz (112) aufweist, der in Verlängerung des Nadelhalteransatzstückes im Inneren der Kapsel angeordnet ist, dass der Versteifungsansatz ein glockenförmiges Unterteil (111) aufweist, welches sich über den ringförmigen Sitz (112) schieben lässt und dass das membranartige Mikrofilter (28) zwischen diesem Sitz und diesem glockenförmigen Unterteil angeordnet ist.

12. Spritze nach irgendeinem der Ansprüche 1, 7 oder 11, dadurch gekennzeichnet, dass das membranartige Mikrofilter (28) durch Eingiessen eingebaut ist, in den Boden der Kapsel, in das Nadelhalteransatzstück, in den Versteifungsansatz oder in den mit dem Boden der Kapsel fest verbundenen ringförmigen Sitz.

13. Spritze nach Anspruch 1, dadurch gekennzeichnet, dass das zu injizierende Medikament unter dem Druck eines in der an einem ihrer Enden geschlossenen Ampulle enthaltenen Gases steht, und dass am Ende des Einspritzvorganges das zumindes teilentspannte Gas das gesamte Volumen zwischen den Innenwänden der Ampulle (11) und dem membranartigen Mikrofilter (28) einnimmt.

14. Spritze nach einem beliebigen der vorhergehenden Ansprüche, dadurch gekennzeichnet, dass die Innenhöhe der Kapsel (12) mindestens gleich der Aussenhöhe der Ampulle (11) ist.

15. Spritze nach einem beliebigen der vorhergehenden Ansprüche, dadurch gekennzeichnet, dass die nutzbare Fläche des Filters kleiner als der kleinste rechtwinklige Querschnitt durch die Ampulle (11) ist.

**FIG. 1**

**FIG. 2**

**FIG. 3**

**FIG. 4**

**FIG. 5**

FIG. 6

FIG. 7

FIG. 8

FIG. 9

FIG. 10

FIG. 11